# EUROPEAN PATENT SPECIFICATION

(11) **EP 0 529 604 B1**
(45) Date of publication and mention of the grant of the patent: **16.10.1996**
(21) Application number: 92114531.4
(22) Date of filing: 26.08.1992
(51) Int. Cl.: G01N 33/50, G01N 30/72, G01N 33/68

(54) **Method of analysing a protein or a peptide**
Verfahren zur Analyse von einem Protein oder einem Peptid
Procédé pour analyser une protéine ou un peptide

(30) Priority: 28.08.1991 JP 217437/91; 15.11.1991 JP 300818/91
(43) Date of publication of application: 03.03.1993
(73) Proprietor: SEIKO INSTRUMENTS INC., Tokyo 136 (JP)
(72) Inventor: Uchida, Tuyoaki, c/o Seiko Instruments Inc., Tojko (JP); Tsugita, Akira, c/o Ishizuka, Kashiwa-shi, Chiba (JP); Takamoto, Keiji c/o Pana-Heights-Renassance 104, Nagareyama-shi, Chiba (JP); Satake, Kazuo, Kawasaki-shi, Kanagawa (JP)
(74) Representative: Sturt, Clifford Mark

(56) References cited:
- EP-A- 0 257 735
- WO-A-89/08835
- CHEMICAL ABSTRACTS, vol. 70, no. 1, 06 January 1969, Columbus, OH (US); N.M. ORLOVSKA et al., p. 68, no. 803j/
- ANALYTICAL BIOCHEMISTRY, vol. 166, no. 2, 01 November 1987; D.H. HAWKE et al., pp. 298-307/
- CHEMISTRY LETTERS, vol. 2, 1992, JP; A.TSUGITA et al., pp. 235-238/

## Description

The present invention relates to a method of analysing the primary structure of a protein or a peptide.

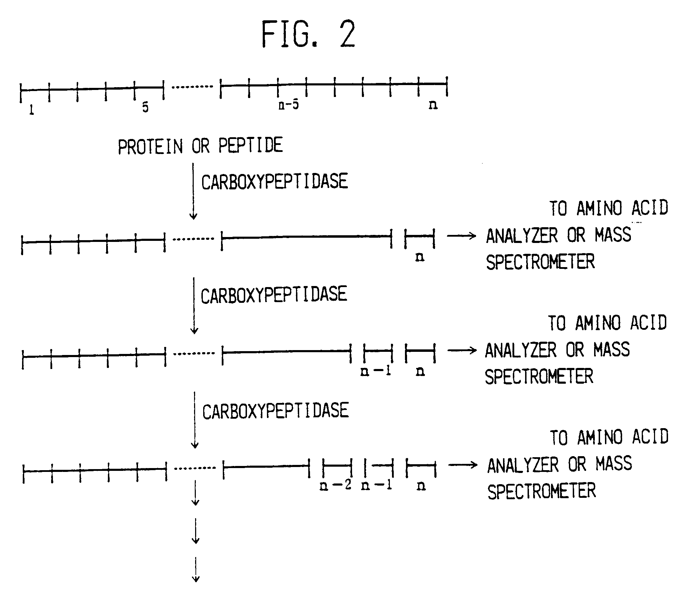

Conventionally, in order to determine an amino acid sequence of a protein or peptide from a carboxy-terminal or C-terminal, as shown in Fig. 2, the protein or peptide is treated with carboxypeptidase. Then, a digested solution is sampled time-sequentially, and the sampled, digested solution is analysed using an amino acid analyser to effect quantitative measurement of free amino acids. Such a method is described in Biochemical Experiment Text, Vol. 1, Chemistry of Protein II, pp 203-211, 1976, edited by the Japanese Biochemistry Society.

There has been reported another method in which the digested solution is analysed using a mass spectrometer to measure a mass of protein or peptide from which amino acids have been digested from the C-terminal. Such a method is disclosed by A. Tsugita, R. van den Broek and M. Pyzybylski in FEBS. Lett. 137, 19(1982).

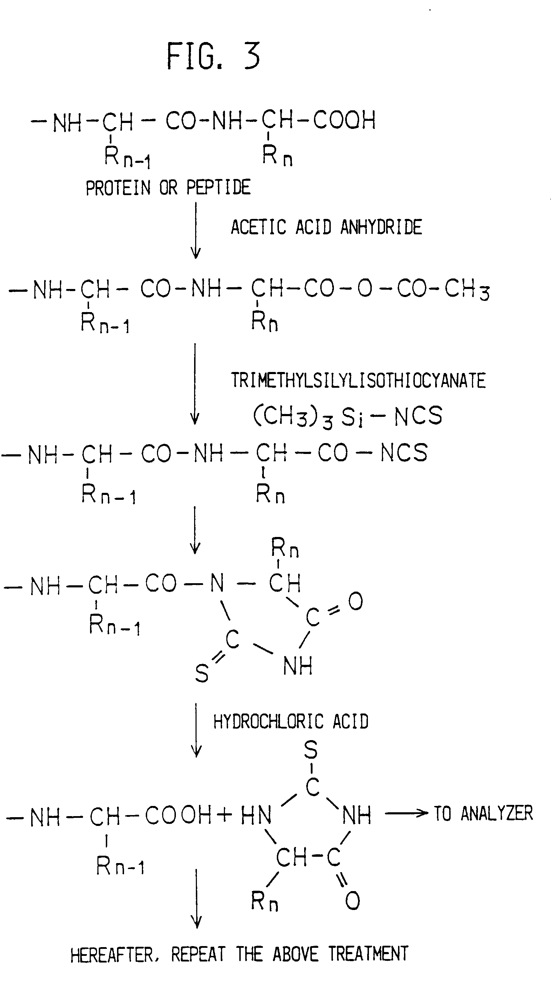

A further method has been reported by D.H.Hawke, H-.W.Lahm, J.E.Shively and C.W.Todd in Anal. Biochem. 166, 298(1987). As shown in Fig. 3, the C-terminal is activated by acetic acid anhydride and is then coupled to trimethylsilylisothiocyanate (TMS-ITC). Thereafter, the protein or peptide is cleaved using hydrochloric acid. This treatment is repeated sequentially to perform the amino acid sequence analysis.

The conventional method utilising carboxypeptidase has a number of disadvantages in that the substrate specificity and the activity of the carboxypeptidase vary according to the C-terminal amino acid or an adjacent amino acid; in that the reagent contains other contaminant enzymes which hinder a precise analysis, and in that an external amino acid is introduced by self-dissociation of the enzyme, thereby reducing the sensitivity of the amino acid analysis. The other conventional method utilising TMS-ITC has the disadvantage that the processing is complicated since three kinds of reagents are applied sequentially and that the repetitive yield rate is rather small, and this method is therefore not in common use.

Therefore, an object of the invention is to provide a simplified method of determining the amino acid sequence of a protein or peptide from a C-terminal without using an enzyme or other complicated organic compounds.

According to a first aspect of the invention, there is provided a method of determining an amino acid sequence of a protein or a peptide, wherein the protein or peptide is sequentially degraded from the C-terminal thereof to give a reaction mixture, and the reaction mixture is analysed to determine the amino acid sequence of the protein or the peptide from the C-terminal, characterised in that the protein or the peptide is treated with a vapour containing an organic acid represented by a general formula CF₃ - (CF₂)ₙ - COOH, where n denotes zero or an integer, and optionally with a reducing agent, so as to produce the reaction mixture. Suitable examples of organic acids are trifluoroacetic acid (n=0), pentafluoropropionic acid (n=1) and heptafluorobutyric acid (n=2). According to this method, the amino acid sequence can be determined from the C-terminal of the protein or peptide by a simplified treatment without utilising an enzyme, while avoiding the above-noted disadvantages.

According to a second aspect of the invention, there is provided a method of determining an amino acid sequence of a protein or a peptide, wherein the protein or the peptide is sequentially degraded from the C-terminal thereof to give a reaction mixture, and the reaction mixture is analysed to determine the amino acid sequence of the protein or the peptide from the C-terminal, characterised in that the protein or the peptide is treated with an anhydride of an organic acid represented by a general formula CF₃ - (CF₂)ₙ - COOH, where n denotes zero or an integer, so as to produce the reaction mixture. Suitable anhydrides include anhydrides of trifluoroacetic acid (n =0), pentafluoropropionic acid (n = 1) and heptafluorobutyric acid (n=2). Using this method, the amino acid sequence can be determined from the C-terminal of the protein or peptide by a simplified treatment without utilising an enzyme or other complicated compounds, while avoiding the above-noted disadvantages.

Figure 1 is a process step chart showing a method according to the first aspect of the invention.

Figure 2 is a process step chart showing a conventional analysis method utilising carboxypeptidase.

Figure 3 is a process step chart showing another conventional analysis method utilising trimethylsilylisothiocyanate.

Figure 4(A) shows an experimental system according to a first aspect of the invention, in which a reducing agent is added into an aqueous solution of an organic acid.

Figure 4(B) shows another experimental system according to a first aspect of the invention, in which a reducing agent is charged into a small test tube while the sample of protein or peptide, to be analysed is held in a separate small test tube.

Figures 5(A)-5(F) show various analysis results of reaction mixtures of a peptide analysed by FAB-MS, where a hexapeptide, sample No. 1, is treated with various vapours containing TFA (Figure 5(B)), PFPA (Figure 5(C)) and HFBA (Figure 5(D)) for 4 hours, and where the hexapeptide is treated with PFPA (Figure 5(E)) and HFBA (Figure 5(F)) for 24 hours, and additionally, the hexapeptide is not treated with organic acid (Figure 5(A)).

Figures 6(A)-6(C) show various analysis results of reaction mixtures of another peptide analysed by FAB-MS, where a nonapeptide, Sample No. 2, is treated with vapours containing HFBA for 4 hours (Figure 6(B)) and for 24 hours (Figure 6(C)), and the nonapeptide is not treated with the organic acid (Figure 6(A)).

Figures 7(A)-7(E) show various analysis results of the hexapeptide, sample No. 1, treated with PFPA at 90^{o}C for 4 hours while varying the concentration of PFPA in the range of 50% to 98%, where the concentration of PFPA is set to 50% (Figure 7(B)), 70% (Figure 7(C)), 90% (Figure 7(D)) and 98% (Figure 7(E)), and the hexapeptide is not treated with organic acid (Figure 7(A)).

Figures 8(A)-8(E) show various analysis results of octapeptide, sample No. 3, treated with HFBA at 90^{o}C for 4 hours while varying the concentration of HFBA in the range of 50% to 98%, where the concentration of HFBA is set to 50% in Figure 8(B), 70% in Figure 8(C), 90% in Figure 8(D) and 98% in Figure 8(E), and the octapeptide is not treated with the organic acid in Figure 8(A).

Figure 9 shows a mass spectrogram indicating an analysis result in which ESI-MS is utilised to analyse a reaction mixture obtained by treating a tricosapeptide, sample No. 4, with PFPA for 2 hours.

Figure 10 is a diagram illustrating data of the Figure 9 analysis results.

Figures 11(A)-11(C) show analysis results in which an amino acid analyser is utilised to analyse reaction mixtures of the hexapeptide, sample No. 1, treated with a vapour containing PFPA for 4 hours (Figure 11(B)) and 24 hours (Figure 11(C)), and is not treated with the organic acid in the case of Figure 11(A).

Figure 12 is a process step chart showing a method according to the second aspect of the invention.

Figure 13 is a diagram showing an experimental system according to a second aspect of the invention.

Figure 14 is a diagram showing an analysis result of a sample peptide, sample No. 5, which is not treated with an acid anhydride.

Figure 15 is a diagram showing an analysis result of a reaction mixture of sample No. 5, treated with the anhydride of PFPA.

Figure 16 is a diagram showing an analysis result of a reaction mixture of sample No. 5, treated with the anhydride of HFBA.

Figure 17 is a diagram showing an analysis result of a reaction mixture of sample No. 5, treated with the anhydride of TFA.

Figure 18 is a diagram showing an analysis result of a sample peptide, sample No. 6, which is not treated with the anhydride of an acid.

Figure 19 is a diagram showing an analysis result of a reaction mixture of sample No. 6, which is treated with an acid anhydride for two hours at -18^{o}C.

Figure 20 is a diagram showing an analysis result of a reaction mixture of sample No. 6 which is treated with an acid anhydride for two hours at 0^{o}C.

Figure 21 is a diagram showing an analysis result of a reaction mixture of sample No. 6 which is treated with an acid anhydride for two hours at - 18^{o}C and is then dried, and which is thereafter treated with water under a weak alkaline condition containing pyridine.

Figure 22 is a diagram showing an analysis result of a peptide, sample No. 4, which is not treated with an acid anhydride.

Figure 23 is a diagram showing an analysis result of a reaction mixture of sample No. 4 which is treated with an acid anhydride for ten minutes at -18^{o}C.

Figure 24 is a diagram showing an analysis result of a reaction mixture of sample No. 4 which is treated with an acid anhydride for 30 minutes at -18^{o}C.

Figure 25 is a diagram showing an analysis result of a reaction mixture of sample No. 4 which is treated with an acid anhydride for one hour at -18^{o}C.

Figure 26 is a diagram showing an analysis result of a reaction mixture of sample No. 4 which is treated with an acid anhydride for 5 hours at -18^{o}C.

The invention is further illustrated with reference to the following Examples.

### EXAMPLES

Hereinafter, a method according to the first aspect of the present invention will be described in conjunction with Examples 1 to 6.

### Example 1

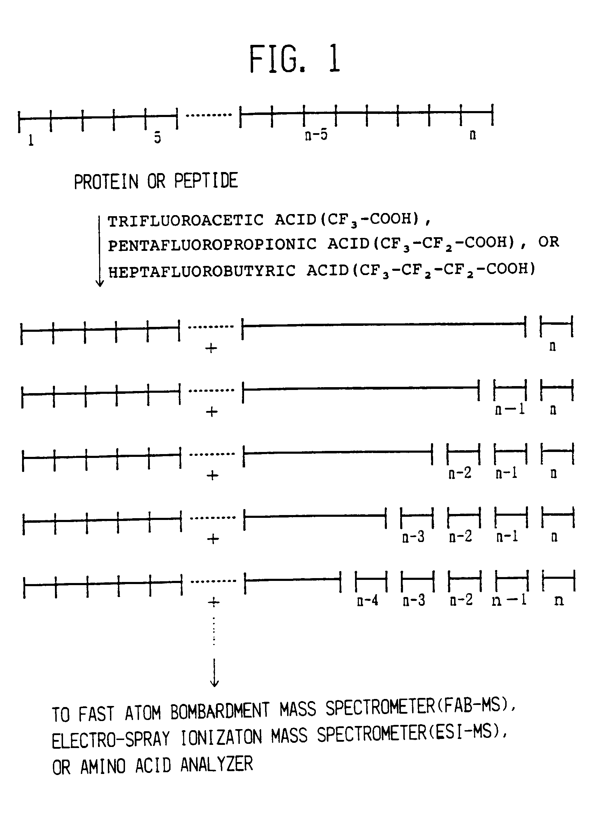

A first experimental method is described in this example. Figure 1 is a process step chart showing the first inventive analysis method. A protein or peptide is treated with trifluoroacetic acid (TFA), pentafluoropropionic acid (PFPA) or heptafluorobutyric acid (HFBA) to cause a successive degradation reaction from the C-terminal. The resulting reaction mixture is analysed by a fast atom bombardment mass spectrometer (FAB-MS) or an electro-spray ionisation mass spectrometer (ESI-MS) to obtain a mass spectrum, or the reaction mixture is processed by an amino acid analyser.

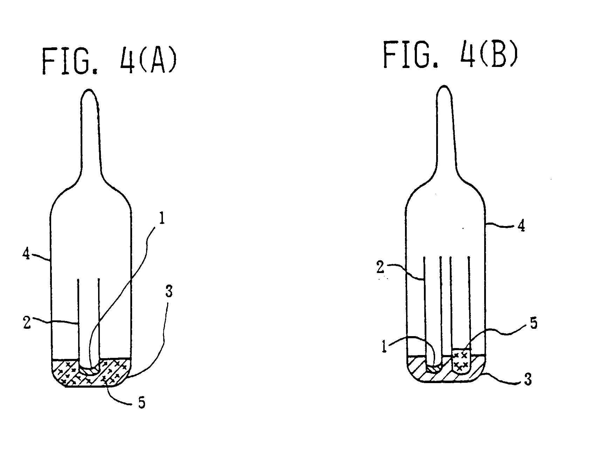

The analysis procedure of the invention is as follows. A sample solution 1 containing a protein or a peptide is charged into a small test tube 2 and is then dried. This small test tube 2 is placed in an outer test tube 4 which contains an aqueous solution 3 of TFA, PFPA or HFBA. In this stage, a reducing agent 5 is added in the aqueous solution (Figure 4(A)). Alternatively, instead of adding the reducing agent into the aqueous solution of the organic acid, the reducing agent 5 may be placed in another separate small test tube, which is then placed together with the small test tube 2 containing the protein or peptide 1 in the outer test tube 4 containing the aqueous solution of the organic acid (Figure 4(B)).

Next, the outer test tube is flame-sealed under a reduced gas pressure state. The double structure of the test tubes is heated. Thereafter, the sealed outer tube is opened and the inner tube is taken out and dried. The dried sample is dissolved into an aqueous solution of acetic acid, and a mixture of glycerol and thioglycerol is added. Thereafter, the sample is analysed by FAB-MS. Alternatively, the sample may be analysed by ESI-MS, after being dissolved into a methanol solution containing acetic acid, and then the sample is introduced into an ESI-MS device.

The condition of the mass spectrography is as follows:

### FAB-MS

- Mass spectrometer :: Model HX110 produced by Japan Electronics Co., Ltd.
- Data processing system ;: DA1000 produced by Japan Electronics Co., Ltd
- Acceleration voltage :: 10kV
- Ion gas species :: Xenon

### ESI-MS

- Mass Spectrometer :: SX-101 produced by Japan Electronics Co., Ltd
- Acceleration voltage :: 10kV
- Ion gas species :: Nitrogen

Alternatively, an amino acid analyser may be utilised for the analysis, and in that case the sample is dissolved into a citric acid buffer solution of pH 2.2 and is then introduced into the analyser. The analyser used may be a model A-5500 produced by Iricakiki Co., Ltd., which performs amino acid analysis by the ninhydrin method with ion exchange chromatography.

### Example 2

In order to demonstrate the invention, an experiment is conducted while selecting, as a sample peptide, hexapeptide, sample No. 1, Leu-Trp-Met-Arg-Phe-Ala. Hereinafter, for example, a segment Leu-Trp-Met is called "peptide 1-3". The various sample peptides are listed in the last part of the specification.

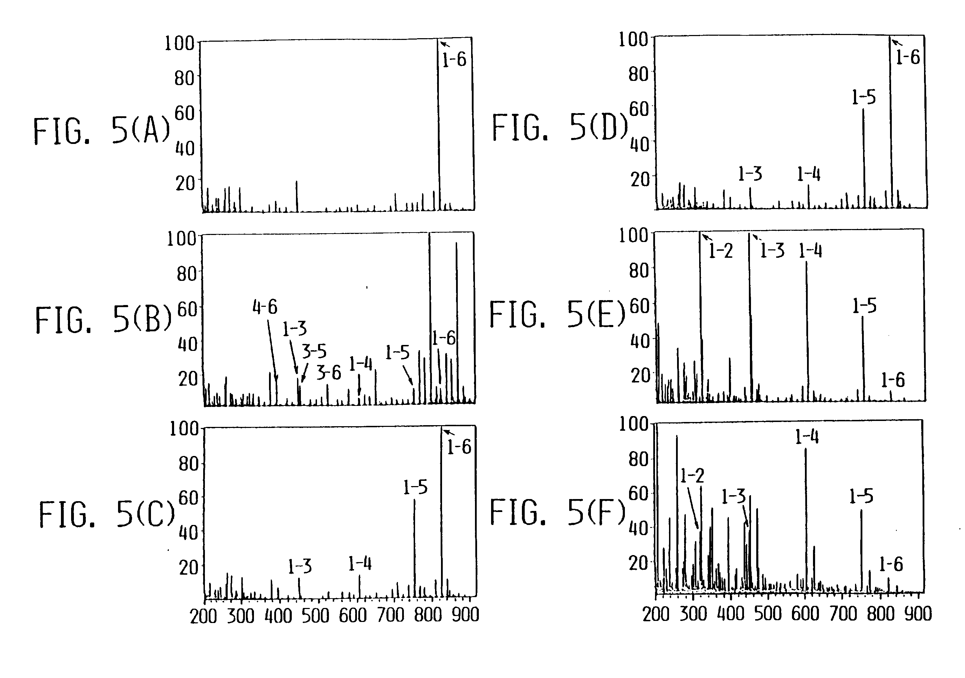

Results of the FAB-MS analysis are shown for the reaction mixture treated with acid vapours containing TFA (Figure 5(B)), PFPA (Figures 5(C), 5(E)) or HFBA (Figures 5(D), 5(F)). In this experiment, the heating temperature is set to 90^{o}C, and the concentration of the respective organic acid solution is set to 90%. Figure 5(A) shows an analysis result of the peptide which is not subjected to the organic acid treatment. When the vapour containing TFA is applied to the sample for 4 hours, the whole peptide is detected as well as degraded peptides 1-5,1-4 and 1-3 in which amino acids are sequentially removed from the C-terminal. However, in this case, subsidiary peptides 4-6, 3-5 and 3-6, produced by non-specific cleavage reactions within the peptide chain, can also be detected. The peptides detained by this method are not limited to those obtained by a sequential cleavage reaction from the C-terminal.

On the other hand, where either of PFPA (Figure 5(C)) and HFBA (Figure 5(D)) is applied to the sample for 4 hours, the peptides detected are mainly the whole peptide 1-6 and fragmental peptides 1-5, 1-4 and 1-3 which are produced by sequential cleavages of the peptide chain from the C-terminal. It is understood that the amino acid sequence can be determined from the C-terminal based on the detection results.

Next, Figures 5(E) and 5(F) show the results obtained when the sample is treated for 24 hours by PFPA and HFBA respectively. In these cases, there are detected cleaved peptides produced by sequential degradation of the peptide chain from the C-terminal in a manner similar to the case where the sample is treated for 4 hours. However, there is detected additionally a fragmental peptide 1-2 which is not observed when the sample is treated for 4 hours. thus, it is understood that a longer sequence of amino acids can be analysed by applying the organic acid for a longer time period.

### Example 3

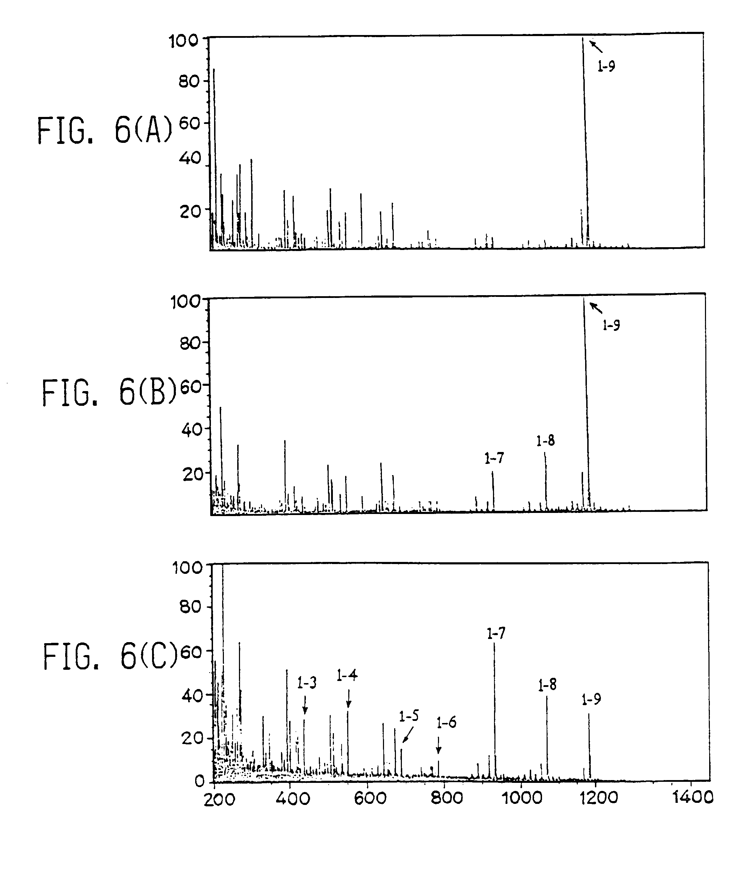

Further, the above described results are confirmed by using another peptide, sample No. 2, nonapeptide Arg-Val-Tyr-Ile-His-Pro-Phe-His-Leu. Figure 6(A) shows an analysis result of the sample peptide which is not treated with the organic acid. Figure 6(B) shows the result of an analysis when the sample is treated with 90% of HFBA for 4 hours at 90^{o}C. The peptides 1-9,1-8 and 1-7 are detected so that the sequence of the last two amino acid units from the C-terminal is determined or identified. When the sample is treated for 24 hours (Figure 6(C)), peptides 1-9, 1-8, 1-7, 1-6, 1-5, 1-4 and 1-3 are detected, so that the sequence of the last six amino acid units is identified.

### Example 4

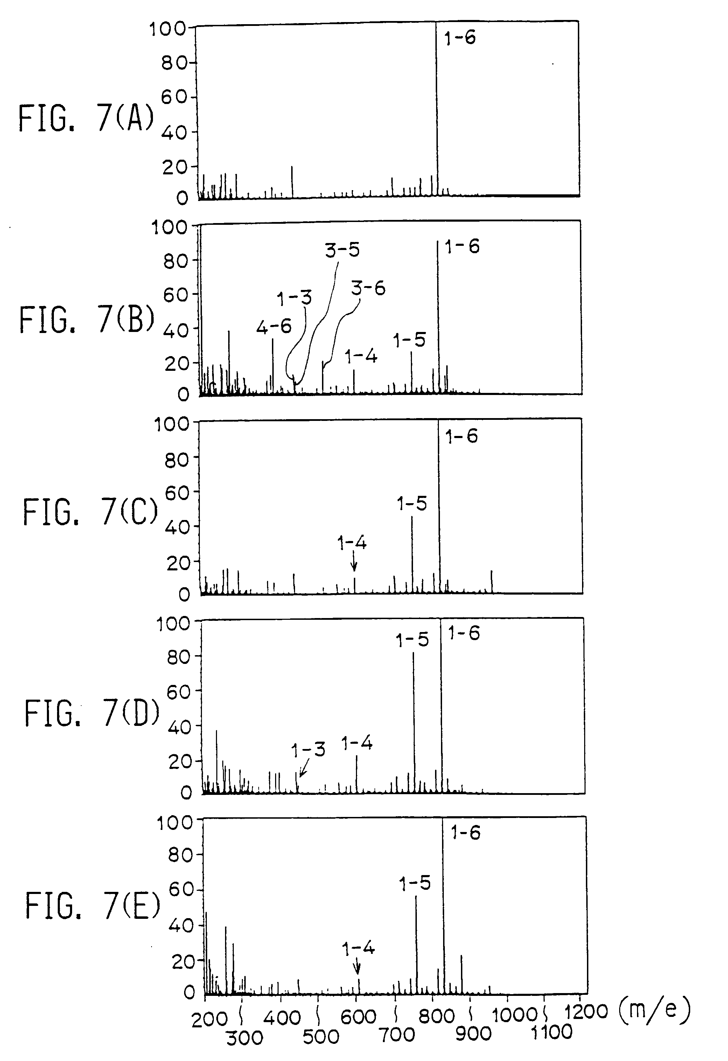

This example shows analysis results obtained when a sample is treated with variable concentrations of the organic acid. Figures 7(A) - 7(E) show the detection results obtained by FAB-MS analysis of a reaction mixture which is obtained by applying PFPA having variable concentration in the range of 50% to 90% to the sample for 4 hours at 90^{o}C. The sample is hexapeptide, sample No. 1, Leu-Trp-Met-Arg-Phe-Ala. Figure 7(A) shows an analysis result when the sample peptide is not treated with the organic acid. Figure 7(B) shows another analysis result where the sample is treated with 50% of PFPA. In a similar manner, Figs. 7(C), 7(D) and 7(E) show different results when the concentration of acid is set to 70%, 90% and 98%, respectively. When the concentration of PFPA is set to 50%, there are detected subsidiary peptide fragments 4-6, 3-5 and 3-6 produced by non-specific dissociation reactions within the peptide chain, which take place at the same time as the sequential cleavage from the C-terminal. It is understood that specific sequential cleavage from the C-terminal occurs most efficiently when the concentration of the organic acid is set to 90%.

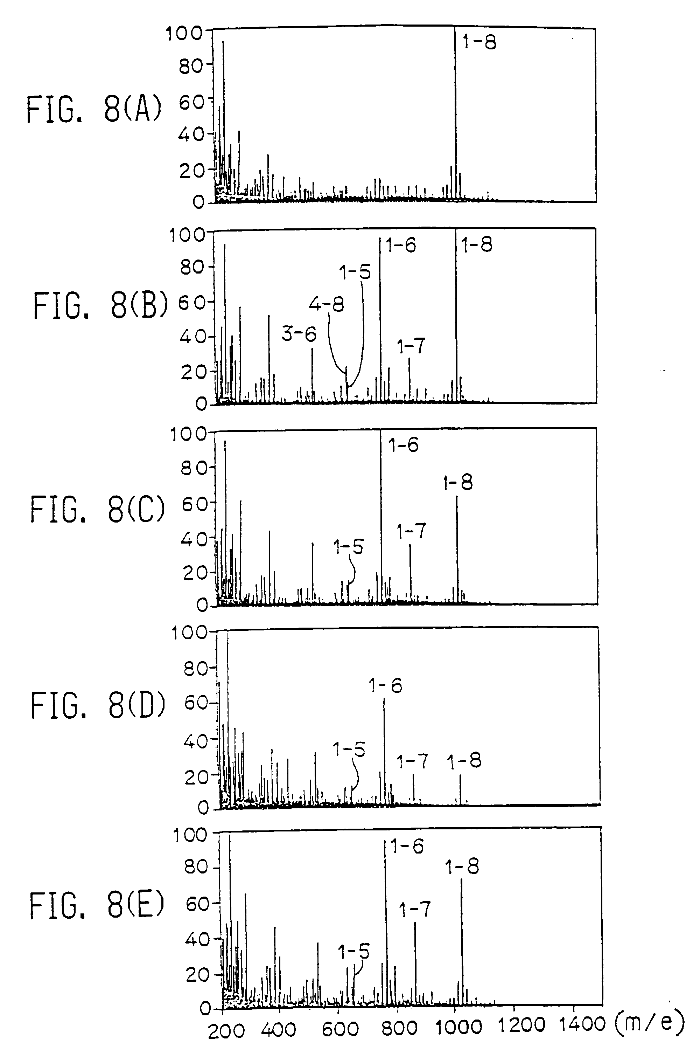

Figures 8(A) - 8(E) shows analysis results obtained when a sample is treated with HFBA for 4 hours at 90^{o}C while varying the concentration in the range of 50% to 98%. The sample is octapeptide, sample No. 3, His-Pro-Phe-His-Leu-Val-Tyr. Figure 8(A) shows an analysis result where the sample is not treated with the organic acid. Figure 8(B) shows another result where the sample is treated with HFBA having a concentration of 50%. In similar manner, Figures 8(C), 8(D) and 8(E) show different results where the concentration is set to 70%, 90% and 98% respectively. When the concentration of HFBA is set to 50%, by-product peptides 4-8 and 3-6 are detected. These peptides are produced by non-specific dissociation reactions within the peptide chain, taking place at the same time as the sequential cleavage from the C-terminal, in a manner similar to the method in which the organic acid is PFPA.

### Example 5

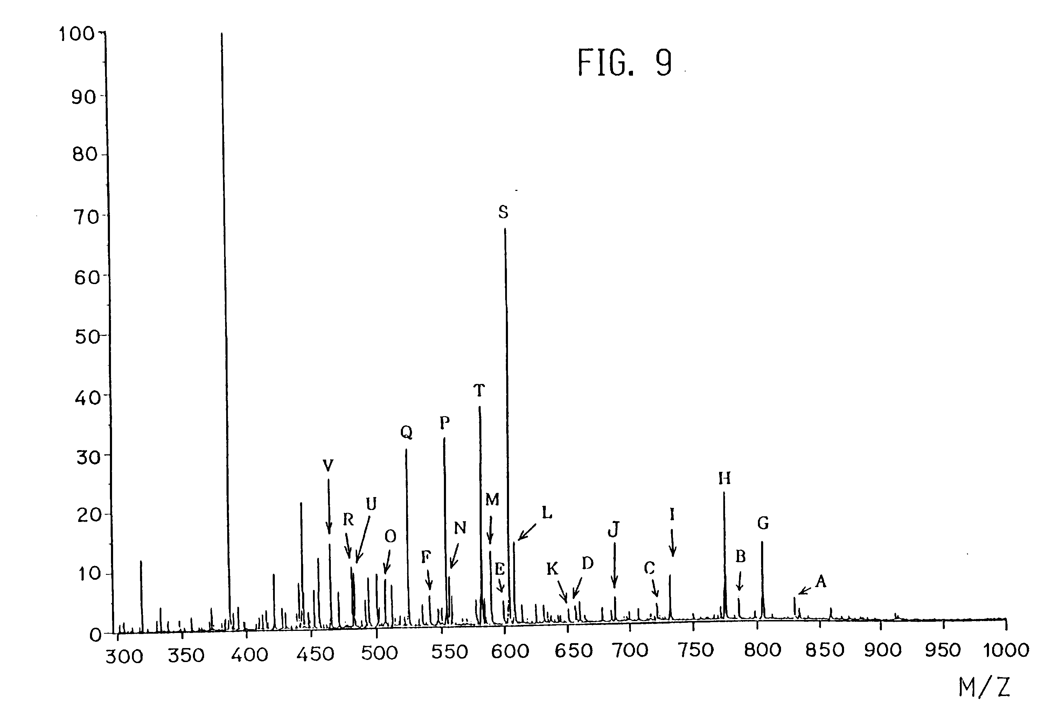

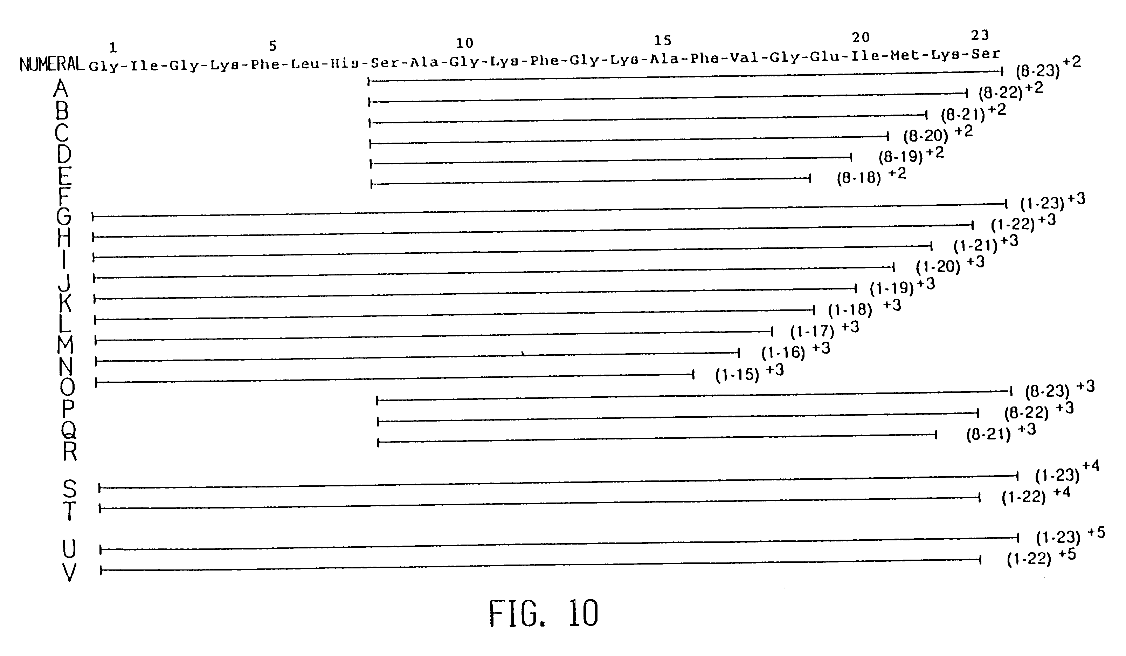

In this example, a reaction mixture is analysed using ESI-MS. Figures 9 and 10 show the analysis result, in which ESI-MS is used to analyse the reaction mixture obtained by applying PFPA in a concentration of 90% for 2 hours at 90^{o}C to tricosapeptide, sample No. 4, Gly-Ile-Gly-Lys-Phe-Leu-His-Ser-Ala-Gly-Lys-Phe-Gly-Lys-Ala-Phe-Val-Gly-Glu-Ile-Met-Lys-Ser. Figure 9 is a mass spectrogram, and Figure 10 is a diagram illustrating the data of the spectrogram. Each reference character in the spectrogram of Figure 9 corresponds to each reference character in Figure 10. For example, a reference numeral (8-23)2+ in Figure10 denotes an ionised peptide 8-23 having double positive charges. Using ESI-MS, ions having various charge units are detected. In this case, ions having triple charges are selectively detected to determine a sequence of the last eight amino acid units from the C-terminal through the ions (1-23)3+ to (1-15)3+.

### Example 6

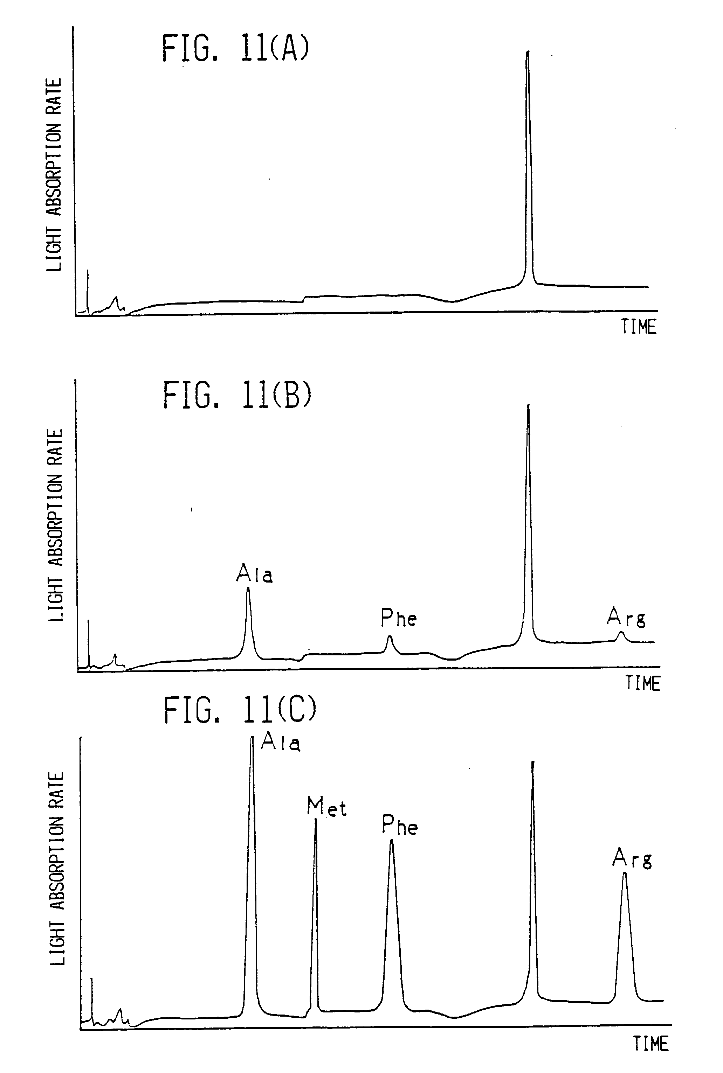

In this example, an amino acid analyser is utilised to analyse a reaction mixture. Figures 11(B) and 11(C) show analysis results when the amino acid analyser is utilised to analyse reaction mixtures which are obtained by applying a vapour containing PFPA for 4 hours and 24 hours, respectively, to hexapeptide, sample No. 1, Leu-Trp-Met-Arg-Phe-Ala, in a manner similar to the method of Example 2. The remaining reaction conditions are similar to those in Example 2, such that the heating temperature is set to 90°C, and the concentration of the organic acid is set to 90%. Figure 11(A) shows an analysis result where the sample is not treated with organic acid. According to a sequence of respective amino acid peaks shown in Figure 11(B), it is determined that the sample has an amino acid sequence of -Arg-Phe-Ala from the C-terminal. Further, according to another sequence of respective amino acid peaks shown in Figure 11(C), it is determined that the sample has an amino acid sequence of -Met-Arg-Phe-Ala from the C-terminal.

The above described Examples 1-6 are summarised as follows. The dried peptide is treated with a vapour which is produced by vapourising an aqueous solution containing 50 to 98% of the organic acid represented by the general formula CF₃ - (CF₂)ₙ - COOH (n is zero or an integer) and optionally with a reducing agent. The resulting reaction mixture is processed by FAB-MS or ESI-MS to obtain a mass spectrum indicative of the whole peptide of the sample and fragmented peptides produced by sequential degradation reactions of amino acids from the C-terminal of the sample peptide. The spectrum is analysed to determine the amino acid sequence of the sample peptide from the C-terminal. Alternatively, the reaction mixture is processed by an amino acid analyser to obtain peak data, which is analysed to determine the amino acid sequence of the sample peptide from the C-terminal.

Hereinafter, the second aspect of the present invention will be described in conjunction with Examples 7 to 11.

### Example 7

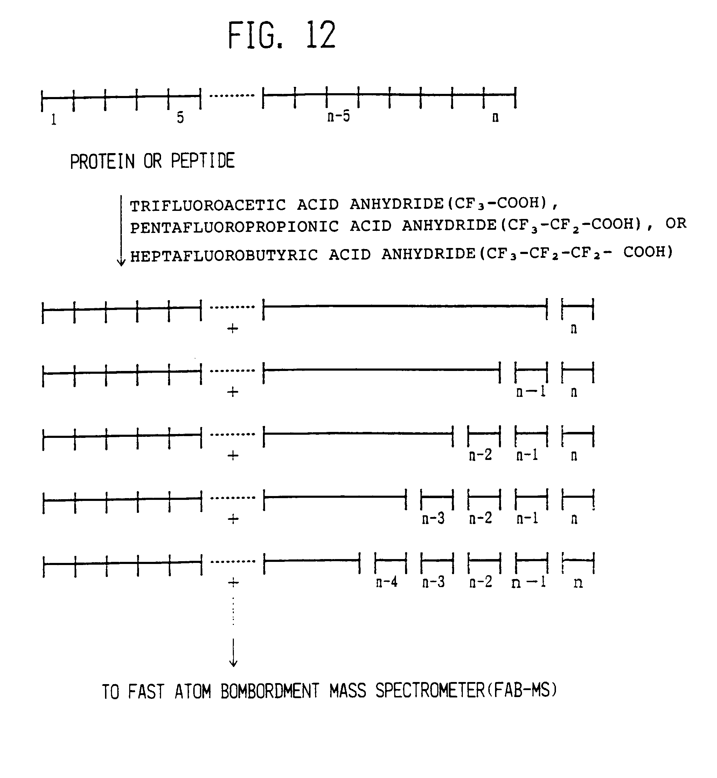

A second experimental method according to the invention is described in this Example. Figure 12 is a process step chart showing the second inventive analysis method. A protein or peptide is treated with an anhydride of trifluoroacetic acid (TFA), pentafluoropropionic acid (PFPA) or heptafluorobutyric acid (HFBA) to cause successive degradation reactions from the C-terminal. The resulting reaction mixture is treated with water and thereafter analysed using a fast atom bombardment mass spectrometer to obtain a mass spectrum.

The analysis procedure according to this aspect of the invention is as follows. Namely, a sample solution 11 containing a protein or a peptide is charged into a small test tube 12, and is then dried. This small test tube is placed in an outer large test tube 14 which contains an acetonitrile solution 13 of an anhydride of TFA, PFPA or HFBA. In this example, the concentration of the anhydride is set at 10%. In this arrangement, the sample solution 11 is not contacted with the anhydride solution 13 of the organic acid. Then, the outer test tube 14 is sealed under vacuum at a temperature of -30°C. This tube is stored at -18°C, as shown in Figurel3. Thereafter, the sealed tube is opened and the contents are dried in vacuo. The dried sample is treated with a vapour of water containing pyridine under a weak alkaline condition. Thereafter, the treated sample is dissolved in dimethyl formamide and is mixed with glycerol, for analysis by FAB-MS. In this process, the aqueous processing treatment is optional.

The condition of mass spectrography is as follows:

### FAB-MS

- Equipment :: Model HX110 produced by Japan Electronics Co., Ltd.
- Ionisation Method :: FAB (Positive)
- Ionisation Gas :: Xenon
- Acceleration Voltage :: 10kV
- Matrix :: Glycerol

### Example 8

In order to demonstrate the present invention, this example is designed to illustrate how an experiment is carried out while selecting, as a sample peptide, an octapeptide, sample No. 5, Lys-Lys-Lys-his-Pro-Asp-Tyr-Ile. In the following description, for example, a fragmental peptide Lys-Lys-Lys-His is denoted by peptide 1-4.

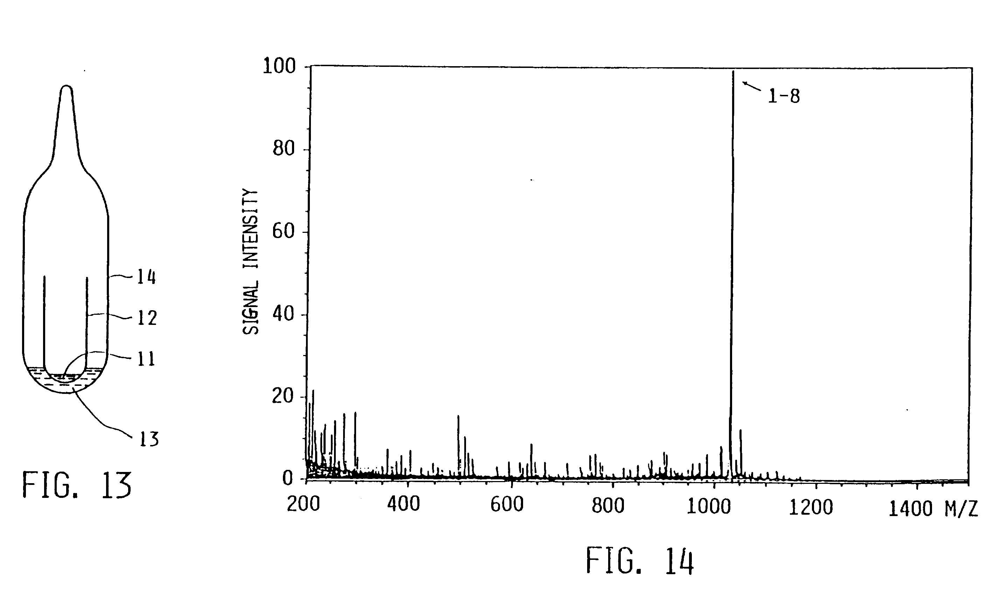

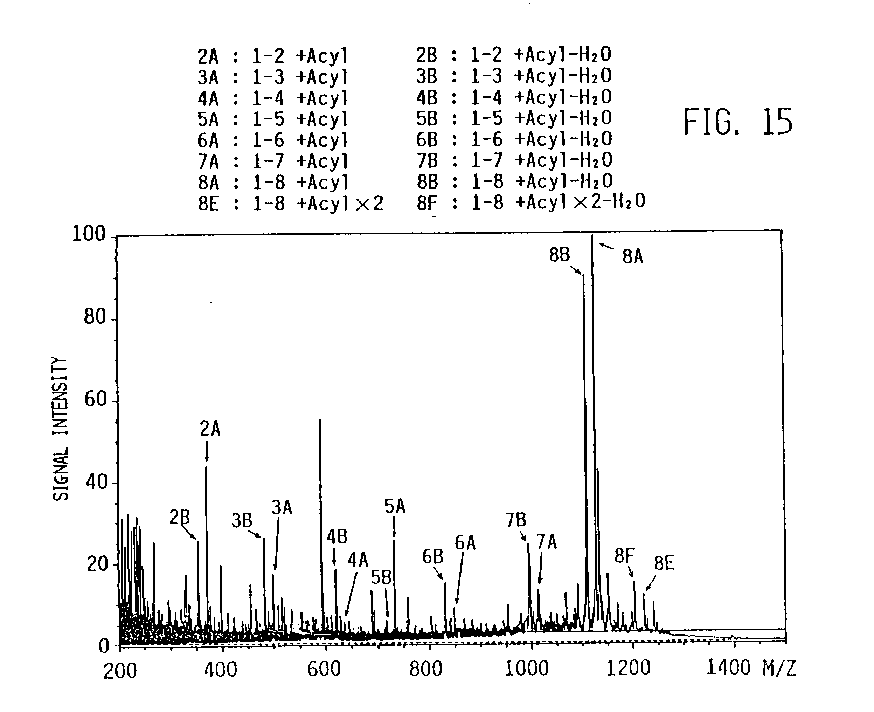

Figures 15, 16 and 17 show analysis results obtained by using FAB-MS to analyse reaction mixtures produced by applying anhydrides of TFA, PFPA and HFBA, respectively. In this experiment, the concentration of the applied organic acid anydrides is set at 10%, and the respective anhydrides are applied for 2 hours at -18^{o}C. Figure 14 shows an analysis result of, sample No. 5, which is not treated with an anhydride of the organic acid, for the purpose of comparison.

Figure 15 shows that the sequence analysis can be achieved from the C-terminal when the anhydride of TFA is applied. Degraded peptides 1-8, 1-7, 1-6, 1-5, 1-4, 1-3 and 1-2 are detected, enabling the sequence of the last six amino acid units from the C-terminal to be determined. In this example, the respective peptide is coupled with TFA and therefore is acylated. For example, an acylated peptide 1-8 is denoted by 1-8+Acyl. Further peaks are observed which correspond to products of degraded peptides from which one molecule of water is lost as indicated by "-H₂O".

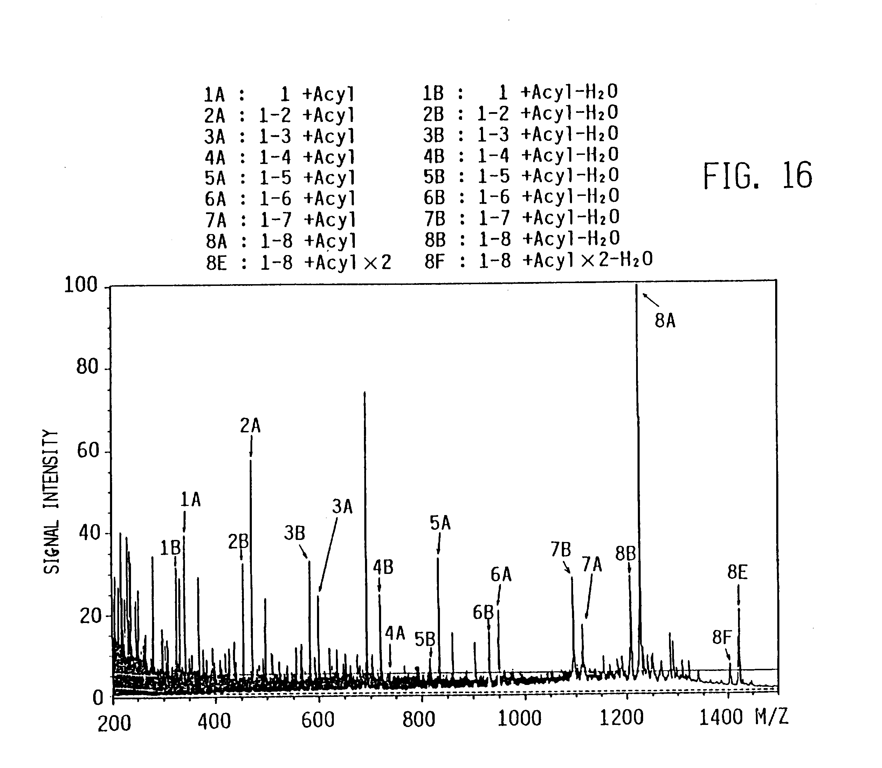

As understood from Figure 16, when the anhydride of PFPA is applied, peptides 1-8, 1-7, 1-6, 1-5, 1-4, 1-3 and 1-2 coupled to PFPA and the amino acid unit of the terminal (hereinafter, referred to as "1 + Acyl") can be detected, thereby determining the complete amino acid sequence from the C-terminal. At the same time, peptide 1-8 coupled with two molecules of PFPA (referred to as "1-8+diAcyl"), can be observed together with various degraded peptides which have lost one molecule of water.

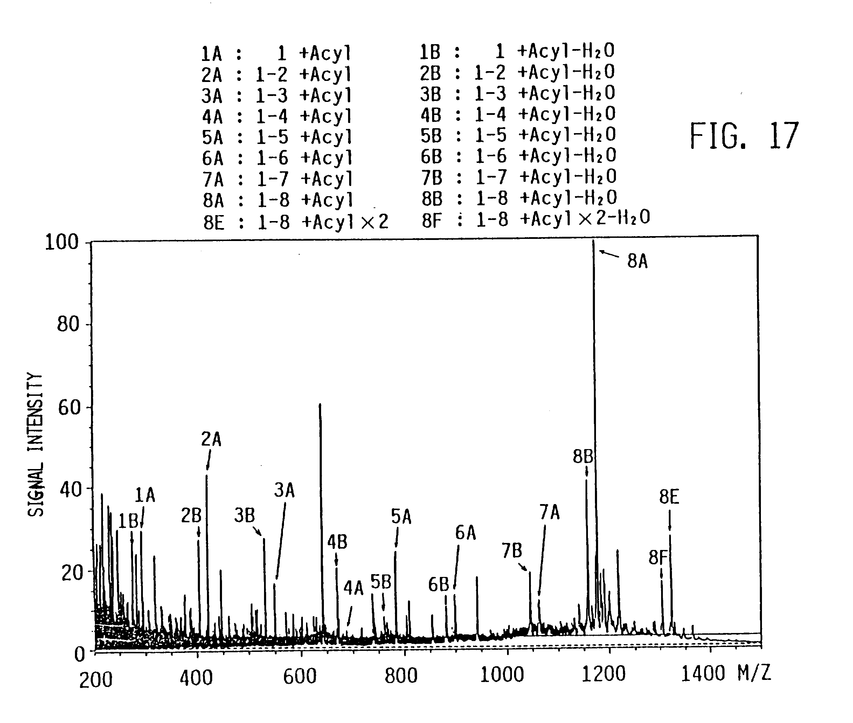

In similar manner, Figure 17 results show that the sequence analysis can be affected from the C-terminal by applying the anhydride of HFBA. Peptides 1-8, 1-7, 1-6, 1-5, 1-4, 1-3, 1-2 and the amino acid unit of the terminal can be detected. The peptides are coupled with HFBA. The complete amino acid sequence from the C-terminal can thus be determined. Peptide 1-8, coupled with two molecules of HFBA can also be observed. Further, there are detected products of cleaved peptides which have lost one molecule of water.

Subsidiary peptides, produced by cleavage of a peptide bond within the peptide chain, are not found in the reaction mixture when this method is used. Therefore, determination of the amino acid sequence may be made by analysis of the peak data.

As described above, a protein or a peptide is treated with an anhydride of the specific organic acids represented by the general formula CF₃ - (CF₂)ₙ - COOH (n is zero or an integer). The reaction products are processed by a mass spectrometer to obtain a mass spectrum. By measuring a mass of the respective products, the amino acid sequence can be determined from the carboxy-terminal of the protein or peptide.

### Example 9

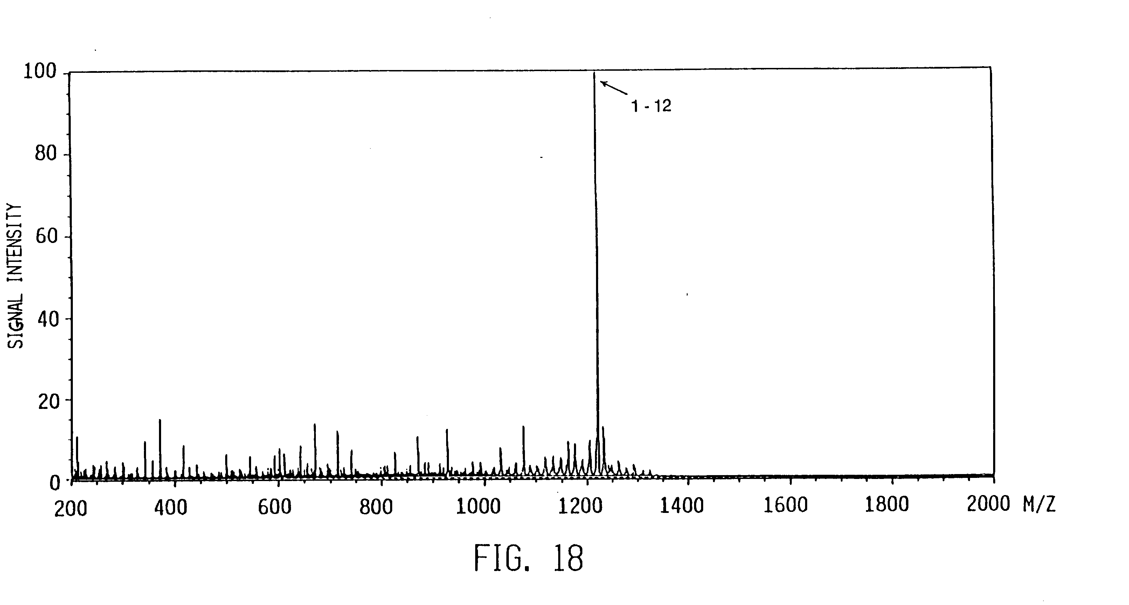

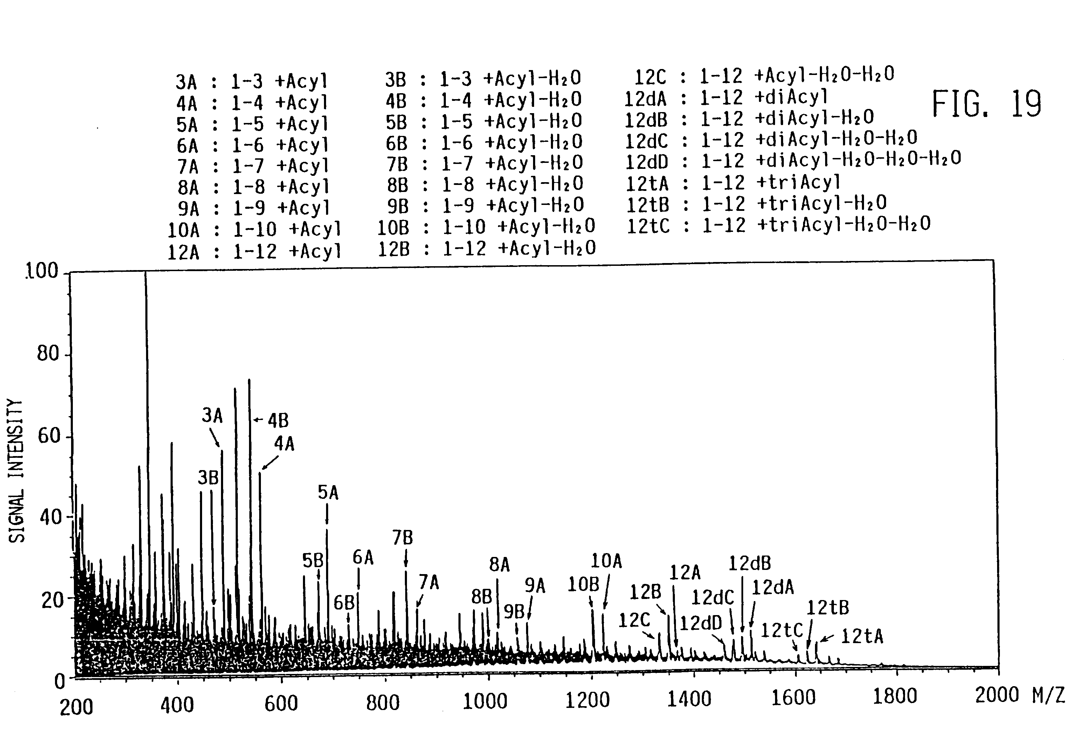

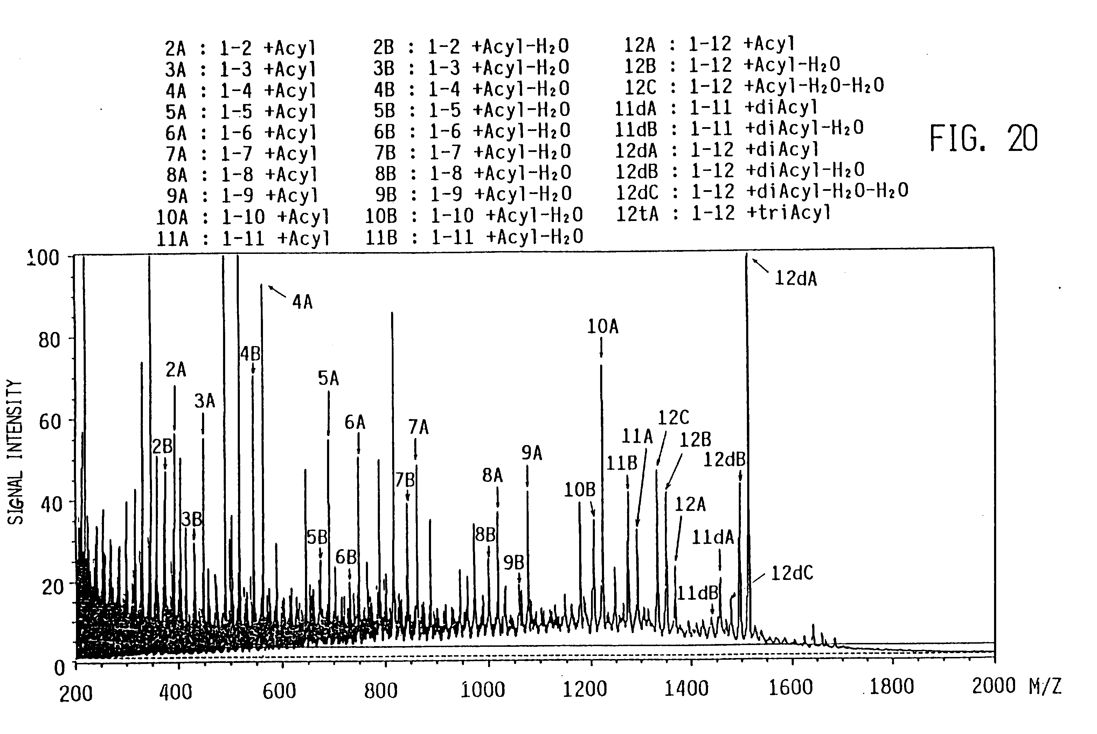

Next, the effect of the reaction temperature when the anhydride of the organic acid is applied, is examined. This example utilised a dodecapeptide, sample No. 6, Ala-Arg-Gly-Ile-Lys-Gly-Ile-Arg-Gly-Phe-Ser-Gly, which is treated with the anhydride of PFPA. Figures 19 and 20 show results obtained by processing, with a mass spectrometer, reaction mixtures treated with the anhydride of PFPA for two hours at temperatures of -18^{o}C and 0^{o}C, respectively. Figure 18 shows the analysis result of the peptide, sample No. 6, which is not treated with the anhydride of the organic acid for the purpose of comparison.

As shown in Figures 19 and 20, peptides 1-12, 1-11, 1-10, 1-9, 1-8, 1-7, 1-6, 1-5, 1-4, 1-3 and 1-2 coupled with PFPA are detected under the respective reaction conditions. It is understood that the sequence of the last ten amino acid units can be determined from the C-terminal under these conditions. At the same time, peptide 1-12, coupled with two molecules of PFPA, is detected.

Further as shown in Figure 20, the peptide 1-11, coupled with two molecules of PFPA is detected when the anhydride of PFPA is applied for two hours at 0^{o}C. Moreover in this case, there are detected more peaks which cannot be identified. It is understood from these results that a number of signals associated with by-products can be reduced while a relative signal intensity related to the objective reaction products can be increased, when the sample is treated at a lower temperature.

### Example 10

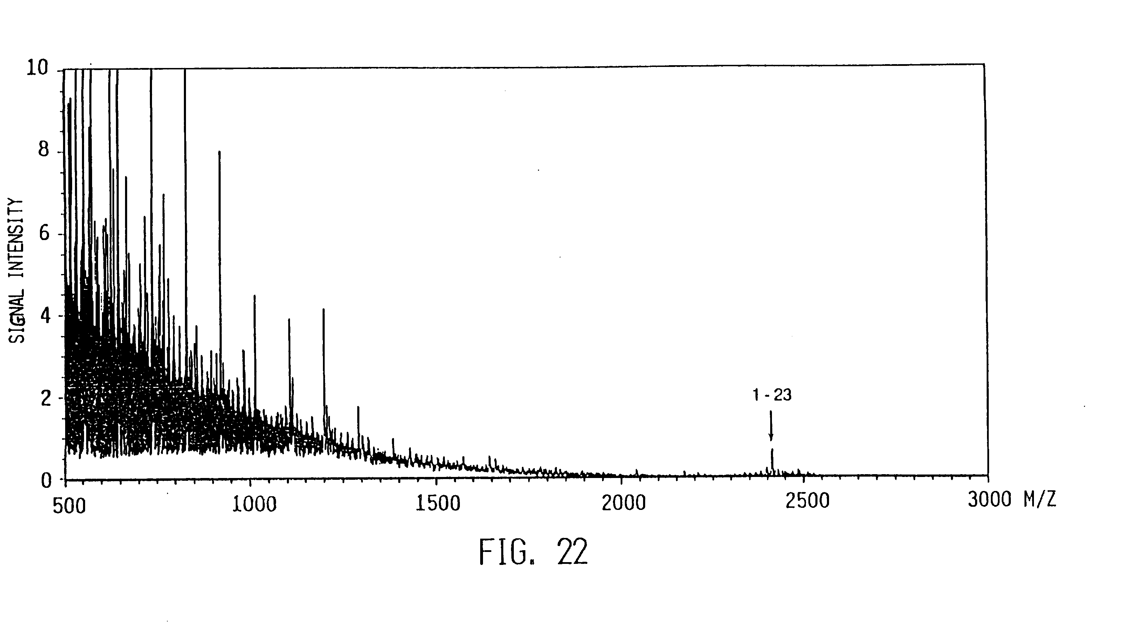

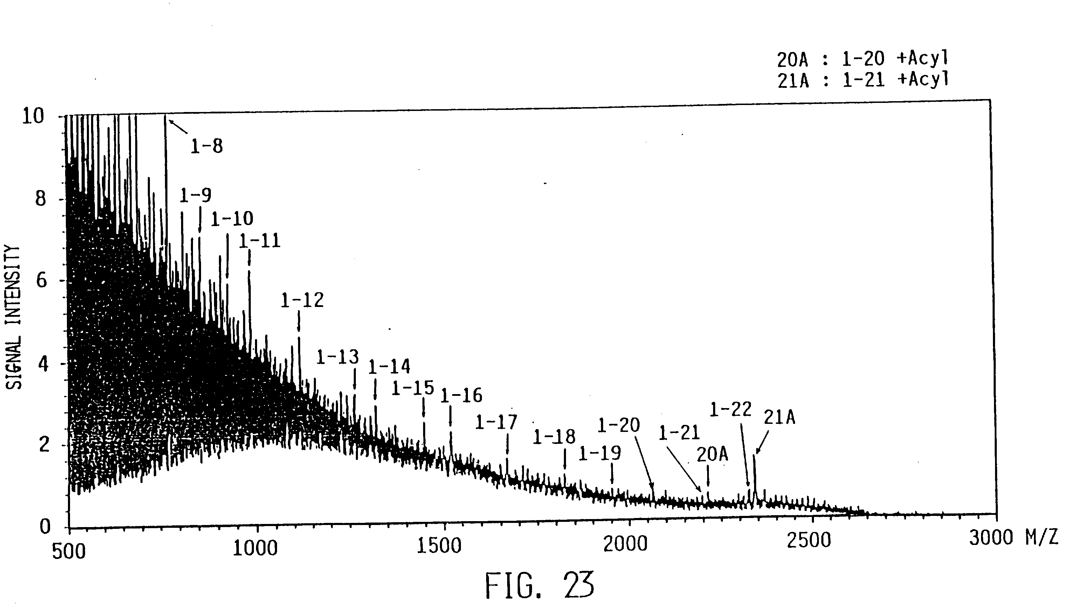

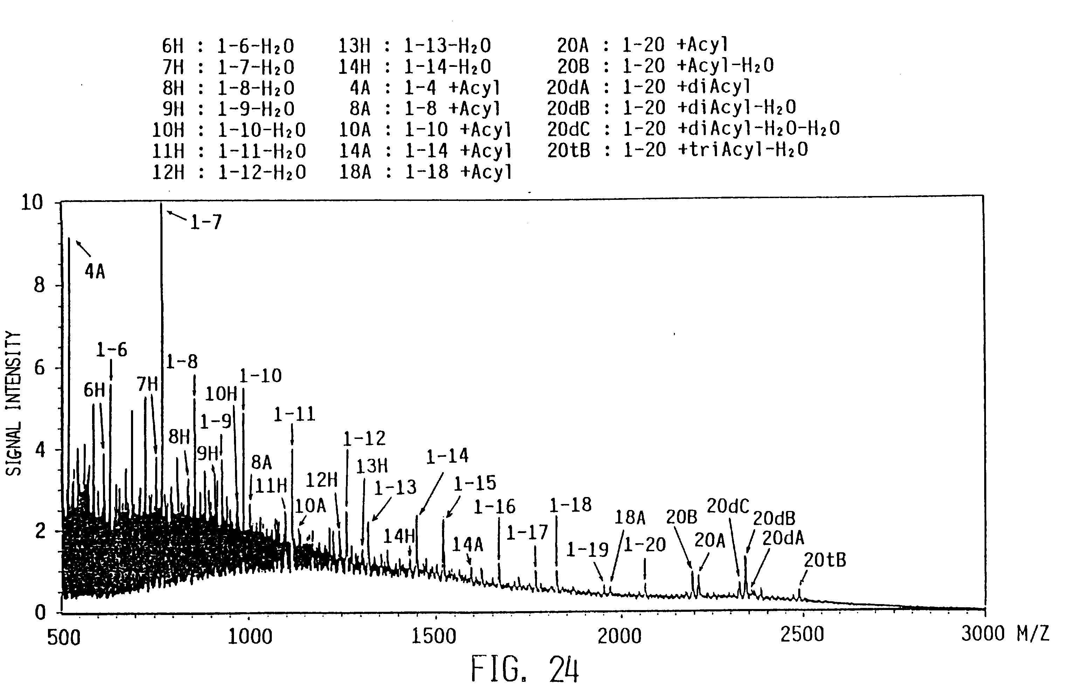

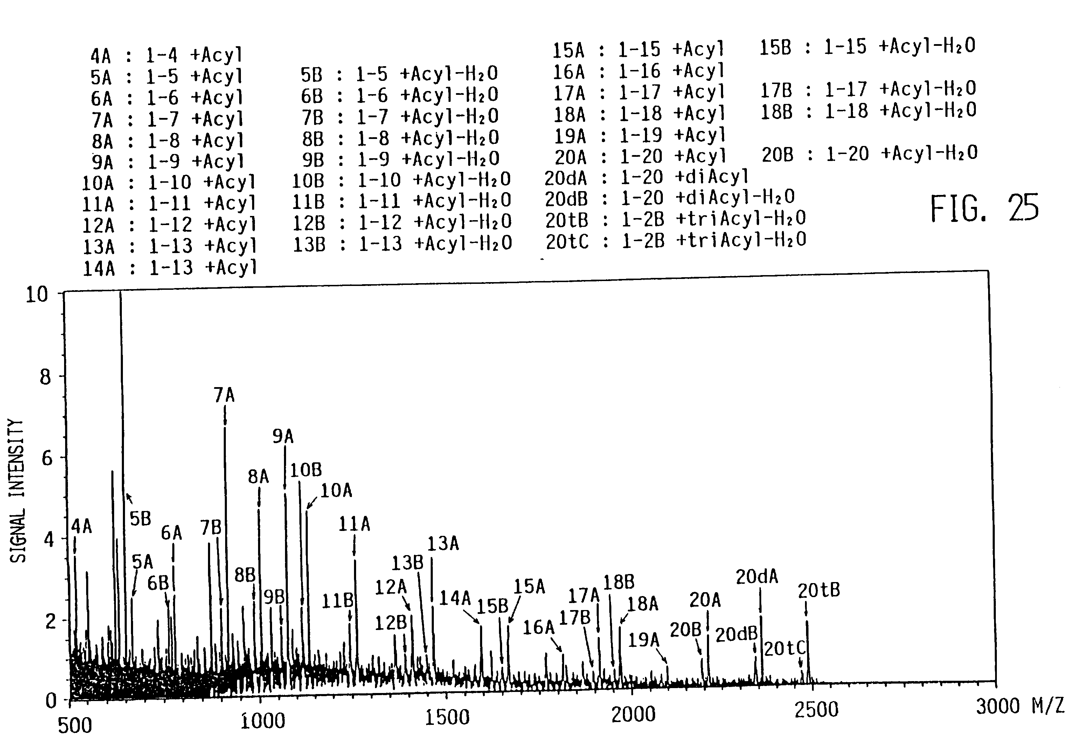

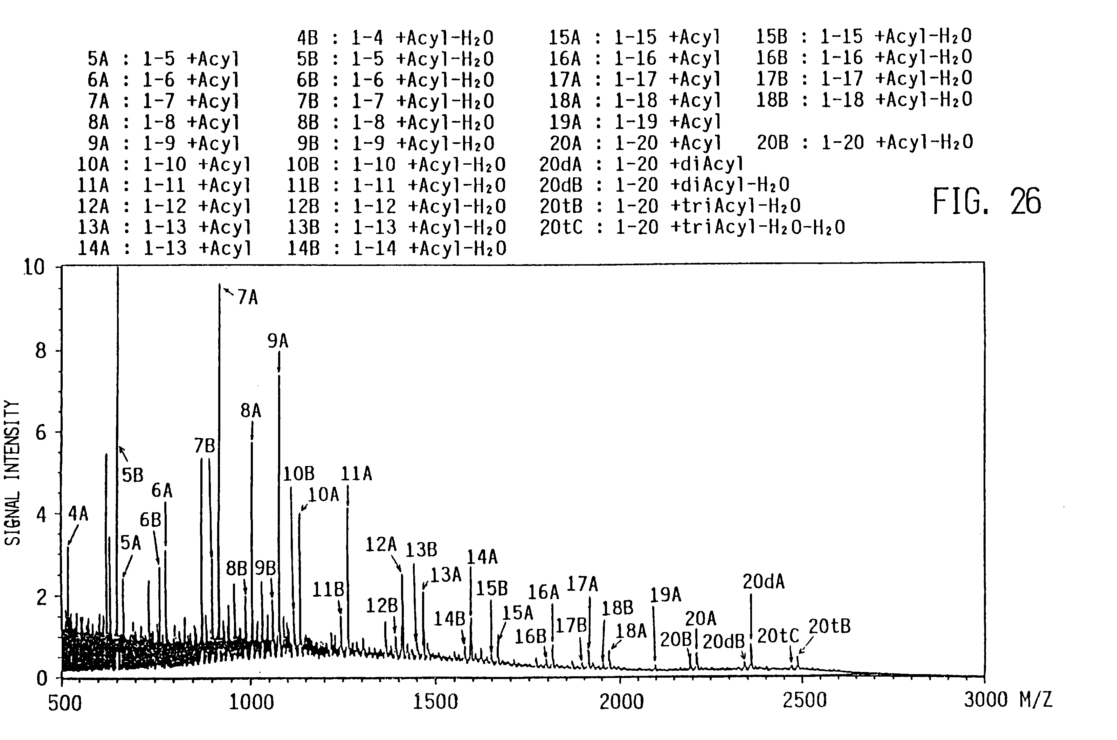

Figures 23 to 26 show analysis results in order to examine the effect of the reaction time interval during which the anhydride of the organic acid is applied, in the determination of the amino acid sequence. This example utilises a sample peptide, sample No. 4, composed of 23 amino acids, which is treated with the anhydride of PFPA at -18^{o}C for 10 minutes (Figure 23), 30 minutes (Figure 24), one hour (Figure 25) and 5 hours (Figure 26). Figure 22 shows the analysis result when sample No. 4 is not treated with the anhydride of the organic acid, for the purpose of comparison.

When the sample is treated for 10 minutes (Figure 23), degraded peptides 1-22, 1-21, 1-20, 1-19, 1-18, 1-17, 1-16, 1-15, 1-14, 1-13, 1-12, 1-11, 1-10, 1-9 and 1-8 are detected, although the intensity of signals indicative of the presence of these sequential peptides is rather weak. Further, it is recognised that acylation occurs due to PFPA in peptides 1-22 and 1-21.

When the sample is treated for 30 minutes (Figure 24) peptides 1-20, 1-19, 1-18, 1-17, 1-16, 1-15, 1-14, 1-13, 1-12, 1-11, 1-10, 1-9, 1-8, 1-7, 1-6, 1-5, and 1-4 are detected. In this case, the acylation by PFPA is not perfect.

Further, when the sample is treated for one hour (Figure 25) and five hours (Figure 26), there are detected sufficient peptides each coupled with PFPA, ie. 1-20, 1-19, 1-18, 1-17, 1-16, 1-15, 1-14, 1-13, 1-12, 1-11, 1-10, 1-9, 1-8, 1-7, 1-6, 1-5, and 1-4. Moreover, the intensity of respective signals is substantially at the same level under these two conditions. It is understood from these results that the anhydride of the organic acid is applied efficiently within 5 hours.

### Example 11

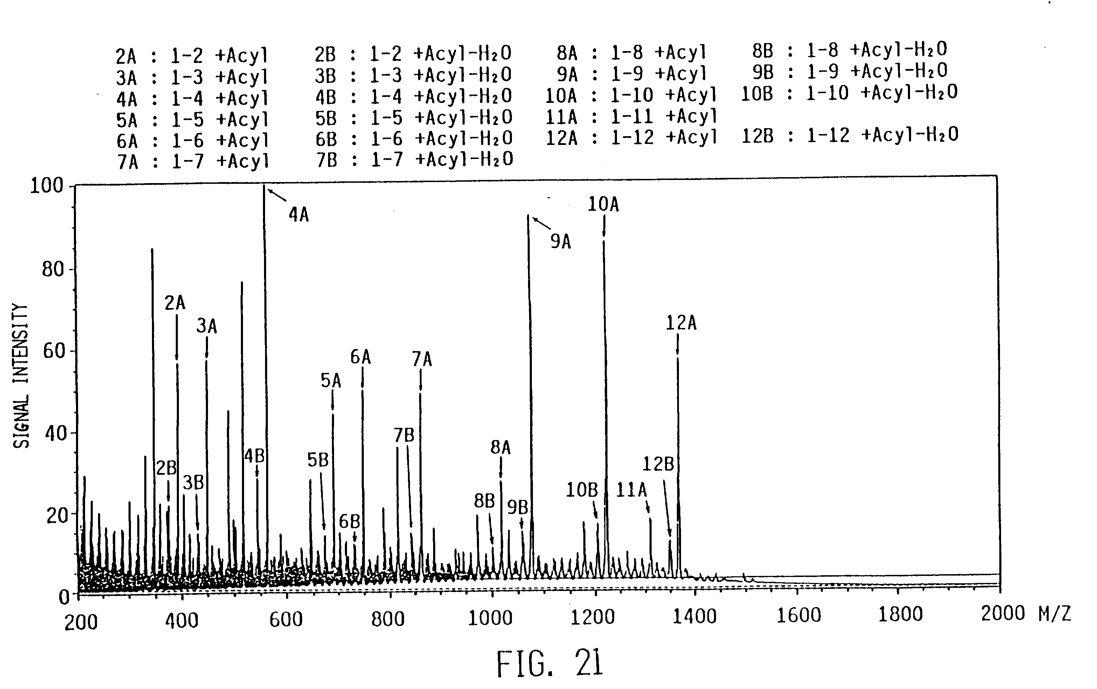

Figure 21 shows the analysis result obtained when water containing pyridine is applied under a weak alkaline condition to a reaction mixture which is obtained by applying the anhydride of PFPA to a dodecapeptide for two hours at - 18^{o}C and then by drying the reaction product. As understood from the comparison to the Figure 20 result, the detection intensity is lowered for sequential peptides 1-12, 1-11, 1-10, 1-9, 1-8, 1-7, 1-6, 1-5, 1-4, 1-3 and 1-2, those of which are coupled with PFPA and those of which have lost one molecule of water. Thus, this treatment can facilitate analysis of the amino acid sequence.

The above described Examples 7-11 are summarised as follows: The dried peptide is treated with an anhydride of the organic acid represented by the general formula CF₃ - (CF₂)ₙ - COOH (n is zero or an integer). The resulting reaction mixture is processed by FAB-MS to obtain a mass spectrum indicative of the whole peptide of the sample and a series of fragmented peptides produced by a sequential degradation reaction of the amino acids from the C-terminal of the sample peptide. The spectrum is analysed to determine the amino acid sequence of the sample peptide from the C-terminal.

The anhydride of the organic acid is applied efficiently for less than 5 hours. The reaction temperature is set at below 0^{o}C so as to suppress progression of subsidiary reactions. Further, after applying the anhydride of the organic acid, the reaction product is preferably treated with water so as to suppress dehydration peaks to thereby facilitate the analysis. Further, in this method, peak data analysis is facilitated for determination of amino acid sequence since no by-product peptides are detected which would be produced by cleavage of a peptide bond within the peptide chain.

### SAMPLE TABLE

- Sample No :: 1
- Length of Sequence :: 6
- Form of Sequence :: Amino acid
- Topology :: Normal chain
- Kind of Sequence :: Peptide
- Sequence ::

Leu-Trp-Met-Arg-Phe-Ala
1 5

- Sample No :: 2
- Length of Sequence :: 9
- Form of Sequence :: Amino acid
- Topology :: Normal chain
- Kind of Sequence :: Peptide
- Sequence ::

Arg-Val-Tyr-Ile-His-Pro-Phe-His-Leu
1 5

- Sample No :: 3
- Length of Sequence :: 8
- Form of Sequence :: Amino acid
- Topology :: Normal chain
- Kind of Sequence :: Peptide
- Sequence ::

His-Pro-Phe-His-Leu-Leu-Val-Tyr
1 5

- Sample No :: 4
- Length of Sequence :: 23
- Form of Sequence :: Amino acid
- Topology :: Normal chain
- Kind of Sequence :: Peptide
- Sequence ::

Gly-Ily-Gly-Lys-Phe-Leu-His-Ser-Ala-Gly-Lys-Phe-
5 10
Gly-Lys-Ala-Phe-Val-Gly-Glu-Ile-Met-Lys-Ser
15 20

- Sample No :: 5
- Length of Sequence :: 8
- Form of Sequence :: Amino acid
- Topology :: Normal chain
- Kind of Sequence :: Peptide
- Sequence ::

Lys-Lys-Lys-His-Pro-Asp-Tyr-Ile
1                    5

- Sample No :: 6
- Length of Sequence :: 12
- Form of Sequence :: Amino acid
- Topology :: Normal chain
- Kind of Sequence :: Peptide
- Sequence ::

Ala-Arg-Gly-Ile-Lys-Gly-Ile-Arg-Gly-Phe-Ser-Gly
1 5 10

## Claims

1. A method of determining an amino acid sequence of a protein or a peptide, wherein the protein or the peptide is sequentially degraded from a C-terminal thereof, to give a reaction mixture, and the reaction mixture is analysed to determine the amino acid sequence of the protein or the peptide from the C-terminal, characterised in that the protein or the peptide is treated with a vapour containing an organic acid represented by a general formula CF₃ - (CF₂)ₙ - COOH where n denotes zero or an integer, and optionally with a reducing agent, so as to produce the reaction mixture.

2. A method according to claim 1, wherein the vapour applied to the protein or the peptide is obtained by vapourising an aqueous solution continuing 50-98% of the organic acid.

3. A method according to claim 1 or claim 2, wherein the step of analysing utilises a mass spectrometer to process the reaction mixture to obtain a mass spectrum thereof as to measure each chemical species contained in the reaction mixture.

4. A method according the claim 1 or claim 2, wherein the step of analysing utilises an amino acid analyser to process the reaction mixture to measure each chemical species contained in the reaction mixture.

5. A method of determining an amino acid sequence of a protein or a peptide, wherein the protein or the peptide is sequentially degraded from a C-terminal thereof, to give a reaction mixture, and the reaction mixture is analysed to determine the amino acid sequence of the protein or the peptide from the C-terminal, characterised in that the protein or the peptide is treated with an anhydride of an organic acid represented by a general formula CF₃ - (CF₂)ₙ -COOH where n denotes zero or an integer, so as to produce the reaction mixture.

6. A method according to claim 5, wherein the anhydride of the organic acid is applied to the protein or the peptide in the form of a solution thereof in a volatile organic solvent

7. A method according to claim 5 or claim 6, wherein the anhydride of the organic acid is applied to the protein or the peptide at a temperature below 0°C.

8. A method according to any of claims 5 to 7, wherein the anhydride of the organic acid is applied to the protein or the peptide for a time interval of less than 5 hours.

9. A method according to any of claims 5 to 8, which further includes the step of treating the reaction mixture with water or steam to produce a reaction product before the step of analysing.

10. A method according to any of claims 5 to 9, wherein the step of analysing utilises a mass spectrometer to obtain a mass spectrum of the reaction product.

## Patentansprüche

1. Verfahren zum Bestimmen einer Aminosäuresequenz eines Proteins oder eines Peptids, worin das Protein oder das Peptid schrittweise von einem terminalen C davon abgebaut wird, um ein Reaktionsgemisch zu ergeben, und worin das Reaktionsgemisch analysiert wird, um die Aminosäuresequenz des Proteins oder des Peptids, von dem terminalen C aus, zu bestimmen, dadurch gekennzeichnet, daß das Protein oder das peptid mit einem Dampf, der eine organische Säure enthält, die durch eine allgemeine Formel CF₃-(CF₂)ₙ-COOH dargestellt wird, worin n Null oder eine ganze Zahl bedeutet, und gegebenenfalls mit einem Reduktionsmittel behandelt wird, um das Reaktionsgemisch zu erzeugen.

2. Verfahren nach Anspruch 1, worin der auf das Protein oder das Peptid angewendete Dampf durch Verdampfen einer wäßrigen Lösung, die 50 bis 98 % der organischen Säure enthält, erhalten wird.

3. Verfahren nach Anspruch 1 oder Anspruch 2, worin der Analyseschritt ein Massenspektrometer verwendet, um das Reaktionsgemisch zu behandeln, um ein Massenspektrum davon zu erhalten, um jede chemische spezies, die im Reaktionsgemisch enthalten ist, zu messen.

4. Verfahren nach Anspruch 1 oder Anspruch 2, worin der Analyseschritt einen Aminosäureanalysator verwendet, um das Reaktionsgemisch zu behandeln, um jede chemische Spezies, die in dem Reaktionsgemisch enthalten ist, zu bestimmen.

5. Verfahren zum Bestimmen einer Aminosäuresequenz eines Proteins oder eines Peptids, worin das Protein oder das Peptid schrittweise von einem terminalen C davon abgebaut wird, um ein Reaktionsgemisch zu ergeben, und worin das Reaktionsgemisch analysiert wird, um die Aminosäuresequenz des Proteins oder des Peptids, von dem terminalen C aus, zu bestimmen, dadurch gekennzeichnet, daB das Protein oder das Peptid mit einem Anhydrid einer organischen Saure, die durch eine allgemeine Formel CF₃-(CF₂)ₙ-COOH dargestellt wird, worin n Null oder eine ganze Zahl bedeutet, behandelt wird, um das Reaktionsgemisch zu erzeugen.

6. Verfahren nach Anspruch 5, worin das Anhydrid der organischen Säure auf das Protein oder das Peptid in der Form einer Losung davon in einem flüchtigen organischen Lösungsmittel angewendet wird.

7. Verfahren nach Anspruch 5 oder Anspruch 6, worin das Anhydrid der organischen Säure auf das Protein oder das Peptid bei einer Temperatur unter 0°C angewendet wird.

8. Verfahren nach einem der Ansprüche 5 bis 7, worin das Anhydrid der organischen Säure auf das Protein oder das Peptid für ein Zeitintervall von weniger als 5 Stunden angewendet wird.

9. Verfahren nach einem der Ansprüche 5 bis 8, welches weiterhin den schritt des Behandelns des Reaktionsgemischs mit Wasser oder Dampf umfaßt, um ein Reaktionsprodukt vor dem Analyseschritt zu erzeugen.

10. Verfahren nach einem der Ansprüche 5 bis 9, worin der Analyseschritt ein Massenspektrometer verwendet, um ein Massenspektrum des Reaktionsprodukts zu erhalten.

## Revendications

1. Procédé de détermination d'une séquence d'acides aminée d'une protéine ou d'un peptide dans lequel la protéine ou le peptide est dégradé séquentiellement à partir de l'une de ses extrémités C pour donner un mélange réactionnel, et le mélange réactionnel est analysé pour déterminer la séquence d'acides aminés de la protéine ou du peptide à partir de l'extrémité C, caractérisé en ce qu'il consiste à traiter la protéine ou le peptide par une vapeur contenant un acide organique représenté par la formule générale CF₃-(CF₂)ₙ - COOH dans laquelle n signifie zéro ou un nombre entier et éventuellement par un agent réducteur de manière à obtenir le mélange réactionnel.

2. Procédé suivant la revendication 1, dans lequel la vapeur appliquée à la protéine ou aux peptides est obtenue en vaporisant une solution aqueuse contenant de 50 à 98 % de l'acide organique.

3. Procédé suivant la revendication 1 ou 2, dans lequel le stade d'analyse utilise un spectromètre de masse pour traiter le mélange réactionnel pour en obtenir un spectre de masse afin de mesurer chaque espèce chimique contenue dans le mélange réactionnel.

4. Procédé suivant la revendication 1 ou 2, dans lequel le stade d'analyse utilise un analyseur d'acides aminés pour traiter le mélange réactionnel afin de mesurer chaque espèce chimique contenue dans le mélange réactionnel.

5. Procédé de détermination d'une séquence d'acide aminé d'une protéine ou d'un peptide, dans lequel la protéine ou le peptide est dégradé séquentiellement à partir de l'une de ces extrémités C pour donner un mélange réactionnel, et le mélange réactionnel est analysé pour déterminer la séquence d'acide aminé de la protéine ou du peptide à partir de l'extrémité C, caractérisé en ce qu'il consiste à traiter la protéine ou le peptide par un anhydride d'un acide organique représenté par la formule générale CF₃ - (CF₂)ₙ - COOH dans laquelle n est zéro ou un nombre entier de manière à produire le mélange réactionnel.

6. Procédé suivant la revendication 5, dans lequel l'anhydride de l'acide organique est appliqué à la protéine ou au peptide sous la forme d'une solution de celui-ci dans un solvant organique volatile.

7. Procédé suivant la revendication 5 ou 6, dans lequel l'anhydride de l'acide de l'acide organique est appliqué à la protéine ou au peptide à une température inférieure à 0°C.

8. Procédé suivant l'une quelconque des revendications 5 à 7, dans laquelle l'anhydride de l'acide organique est appliqué à la protéine ou au peptide pendant un laps de temps inférieur à 5 heures.

9. Procédé suivant l'une quelconque des revendications 5 à 8, qui inclue en outre le stade de traitement du mélange réactionnel à l'eau ou à la vapeur pour produire un produit de réaction avant le stade d'analyse.

10. Procédé suivant l'une quelconque des revendications 5 à 9, dans lequel le stade d'analyse utilisé un spectromètre de masse pour obtenir un spectre de masse du produit de réaction.
